# EUROPÄISCHE PATENTSCHRIFT

(11) **EP 0 682 017 B1**
(45) Veröffentlichungstag und Bekanntmachung des Hinweises auf die Patenterteilung: **11.08.1999**
(21) Anmeldenummer: 95106596.0
(22) Anmeldetag: 02.05.1995
(51) Int. Cl.: C07D 217/26, C07D 217/02, C07D 215/56, C07D 215/54, C07D 215/48, C07C 279/22, A61K 31/47, A61K 31/165

(54) **Substituierte bizyklische Heteroaroylguanidine, Verfahren zu ihrer Herstellung, ihre Verwendung als Medikament oder Diagnostikum sowie sie enthaltendes Medikament**
Substituted bicycle heteroaroylguanidines, their preparation and their application in medicaments and diagnostic agents
Hétéroaroylguanidines bicycliques substituées, leur préparation et leur application dans des médicaments et du agents diagnostiques

(30) Priorität: 05.05.1994 DE 4415873
(43) Veröffentlichungstag der Anmeldung: 15.11.1995
(73) Patentinhaber: HOECHST AKTIENGESELLSCHAFT, 65929 Frankfurt am Main (DE)
(72) Erfinder: Schwark, Jan-Robert, Dr., D-65929 Frankfurt (DE); Kleemann, Heinz-Werner, Dr., D-65474 Bischofsheim (DE); Lang, Hans-Jochen, Dr., D-65719 Hofheim (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 556 672
- EP-A- 0 556 673
- EP-A- 0 556 674
- EP-A- 0 590 455
- EUR. J. MED. CHEM.--CHIM. THER. (EJMCA5,02235234);85; VOL.20 (4); PP.381-3, DAVIS H L ET AL '2,3-Disubstituted 1,8-naphthyridines as potential diuretic agents. 3. 4- and 7-Phenyl derivatives'
- J. MED. CHEM. (JMCMAR);77; VOL.20 (6); PP.838-41, HAWES E M ET AL '2,3-Disubstituted 1,8-naphthyridines as potential diuretic agents. 2. 5,7-Dimethyl derivatives'
- J. MED. CHEM. (JMCMAR);77; VOL.20 (1); PP.124-8, GORECKI D K J ET AL '2,3-Disubstituted 1,8-naphthyridines as potential diuretic agents'
- J. MED. CHEM. (JMCMAR);73; VOL.16 (7); PP.849-53, HAWES E M ET AL '2,3-Disubstituted 1,6-naphthyridines as potential diuretic agents'

## Beschreibung

Substituierte bizyklische Heteroaroylguanidine, Verfahren zu ihrer Herstellung, ihre Verwendung als Medikament oder Diagnostikum sowie sie enthaltendes Medikament

Die Erfindung betrifft bizyklische Heteroaroylguanidine der Formel I 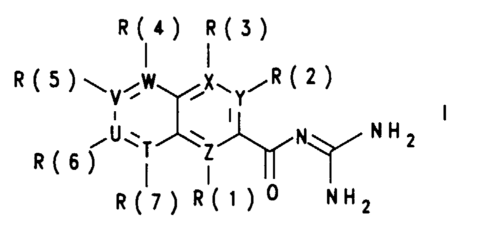 worin bedeuten:
- T, U, V, W, X, Y und Z: unabhängig voneinander Stickstoff oder Kohlenstoff;
jedoch mit der Einschränkung,
daß nur eine der Positionen T, U, V, W, X, Y und Z Stickstoff ist,
und
daß T, U, V, W, X, Y und Z keinen Substituenten tragen, wenn sie Stickstoff sind,
und
daß T, U, V, W, X, Y und Z nicht gleichzeitig Kohlenstoff sind;
und
daß R(3), R(4), R(5), R(6) und R(7) nicht alle gleichzeitig Wasserstoff sind, wenn
a) T, U, V, W, X und Z gleich Kohlenstoff und Y gleich Stickstoff sind, oder
b) T, U, V, W, Y und Z gleich Kohlenstoff und X gleich Stickstoff sind;
- R(1) und R(2): unabhängig voneinander Wasserstoff, F, Cl, Br, I, (C₁-C₃)-Alkyl, (C₁-C₃)-Perfluoralkyl, OR(8) oder NR(8)R(9);
R(8) und R(9) unabhängig voneinander Wasserstoff oder (C₁-C₃)-Alkyl;
oder
R(8) und R(9) gemeinsam 4 oder 5 Methylengruppen, von denen eine CH₂-Gruppe durch Sauerstoff, Schwefel, NH, N-CH₃ oder N-Benzyl ersetzt sein kann,
- R(3), R(4), R(5), R(6) und R(7): unabhängig voneinander Wasserstoff, F, Cl, Br, I, -C≡N, CF₃, CH₃SO₂ oder CH₃CO;
oder
- R(3), R(4), R(5), R(6) und R(7): unabhängig voneinander (C₁-C₄)-Alkyl oder -C_{aI}H_{2aI}R(18);
al Null oder 1;
R(18) Phenyl, welches nicht substituiert oder substituiert ist mit 1 - 3 Substituenten ausgewählt aus der Gruppe bestehend aus F, Cl, CF₃, Methyl, Methoxy und NR(19a)R(19b);
R(19a) und R(19b) H, CH₃ oder CF₃;
oder
- R(3), R(4), R(5), R(6) und R(7): unabhängig voneinander (C₁-C₉)-Heteroaryl, das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
- R(3), R(4), R(5), R(6) und R(7): unabhängig voneinander SR(29), -OR(30), -NR(31)R(32) oder -CR(33)R(34)R(35);
R(29), R(30), R(31) und R(33) unabhängig voneinander -CₐH₂ₐ-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null, 1 oder 2;
R(32), R(34) und R(35) unabhängig voneinander wie R(29) definiert oder Wasserstoff, CH₃ oder CF₃;
oder
- R(3), R(4), R(5), R(6) und R(7): unabhängig voneinander NR(84a)R(85), OR(84b) oder -CₙH₂ₙ-R(84d);
n Null oder 1;
R(84d) Phenyl, welches nicht substituiert ist oder substituiert mit 1 - 3 Substituenten ausgewählt aus der Gruppe bestehend aus F, Cl, CF₃, Methyl, Methoxy und NR(16)R(17);
R(16) und R(17) Wasserstoff oder CH₃;
R(84a), R(84b) und R(85) unabhängig voneinander H, (C₁-C₄)-Alkyl, CF₃ oder (CH₂)ₐₓ-R(84g);
ax Null oder 1;
R(84g) Phenyl, welches nicht substituiert ist oder substituiert mit 1 - 3 Substituenten ausgewählt aus der Gruppe bestehend aus F, Cl, CF₃, Methyl, Methoxy und NR(84u)R(84v);
R(84u) und R(84v) Wasserstoff oder CH₃;
oder
- R(84a) und R(85): gemeinsam 4 oder 5 Methylengruppen, von denen eine CH₂-Gruppe durch Sauerstoff, Schwefel, NH, N-CH₃ oder N-Benzyl ersetzt sein kann,
sowie deren pharmazeutisch verträgliche Salze.

Bevorzugt sind Verbindungen der Formel I, in denen bedeuten:
- T, U, V, W, Y und Z: Kohlenstoff;
- X: Stickstoff;
jedoch mit der Einschränkung,
daß X keinen Substituenten trägt,
und
daß R(4), R(5), R(6) und R(7) nicht alle gleichzeitig Wasserstoff sind; R(1) und R(2)
unabhängig voneinander Wasserstoff, F, Cl, CH₃, CF₃, OH, OCH₃, NH₂; R(4), R(5), R(6) und R(7)
unabhängig voneinander Wasserstoff, F, Cl, Br, I, -C≡N, CF₃, CH₃SO₂ oder CH₃CO,
mit der Einschränkung, daß sie nicht alle gleichzeitig Wasserstoff sind;
oder
- R(4), R(5), R(6) und R(7): unabhängig voneinander (C₁-C₄)-Alkyl oder -C_{aI}H_{2aI}R(18);
al Null oder 1;
R(18) Phenyl, welches nicht substituiert oder substituiert ist mit 1 - 3 Substituenten ausgewählt aus der Gruppe bestehend aus F, Cl, CF₃, Methyl, Methoxy und NR(19a)R(19b);
R(19a) und R(19b) H, CH₃ oder CF₃;
oder
- R(4), R(5), R(6) und R(7): unabhängig voneinander SR(29) oder -OR(30);
R(29) und R(30) unabhängig -CₐH₂ₐ-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null oder 1;
oder
- R(4), R(5), R(6) und R(7): unabhängig voneinander NR(84a)R(85), OR(84b) oder -CₙH₂ₙ-R(84d);
n Null oder 1;
R(84d) Phenyl, welches nicht substituiert ist oder substituiert mit 1 - 3 Substituenten ausgewählt aus der Gruppe bestehend aus F, Cl, CF₃, Methyl, Methoxy und NR(16)R(17);
R(16) und R(17) Wasserstoff oder CH₃;
- R(84a), R(84b) und R(85): unabhängig voneinander H, (C₁-C₄)-Alkyl, CF₃ oder (CH₂)ₐₓ-R(84g);
ax Null oder 1;
R(84g) Phenyl, welches nicht substituiert ist oder substituiert mit 1 - 3 Substituenten ausgewählt aus der Gruppe bestehend aus F, Cl, CF₃, Methyl, Methoxy und NR(84u)R(84v);
R(84u) und R(84v) Wasserstoff oder CH₃.
sowie deren pharmazeutisch verträgliche Salze.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bizyklischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(7) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkyl- und Perfluoralkylreste können sowohl geradkettig wie verzweigt vorliegen.

Die Erfindung betrifft weiterhin ein Verfahren zum Herstellen einer Verbindung I nach Anspruch 1, dadurch gekennzeichnet, daß man eine Verbindung der Formel II 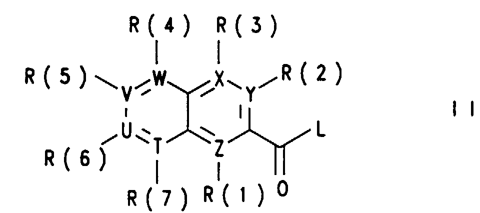 in der R(1) bis R(7) und T, U, V, W, X, Y und Z wie in Anspruch 1 definiert sind und worin L für eine leicht nucleophil substituierbare Fluchtgruppe steht, mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.
Neben den Carbonsäurechloriden der Formel II (L = Cl) lassen sich auch weitere aktivierte Säurederivate der Formel II in an sich bekannter Weise direkt aus den zugrundeliegenden Heteroarylcarbonsäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1,351-367 (1962)], die gemischten Anhydride II mit Cl-COOC₂H₅ oder Tosylchlorid in Gegenwart von Triethylamin in einem inerten Lösungsmittel, wie auch die Aktivierungen von Heteroarylcarbonsäuren mit Dicyclohexylcarbodiimid (DCC) oder mit O-[(Cyano(ethoxycarbonyl)-methylen)amino)-1,1,3,3-tetramethyluroniumtetrafluoroborat] ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren, aber inerten organischen Lösungsmittel. Dabei haben sich bei der Umsetzung der Heteroarylcarbonsäuremethylester (II, L=OMe) mit Guanidin Methanol, Isopropanol oder THF zwischen 20°C und Siedetemperatur dieser Lösungsmittel bewährt. Bei den meisten Umsetzungen von Verbindungen II mit salzfreien Guanidin wurde vorteilhaft in inerten Lösungsmitteln wie THF, Dimethoxyethan, Dioxan oder Isopropanol gearbeitet. Aber auch Wasser kann als Lösungsmittel dienen.

Wenn L = Cl bedeutet, arbeitet man vorteilhaft unter Zusatz eines Säurefängers, z.B. in Form von überschüssigen Guanidin zur Abbindung der Halogenwasserstoffsäure.

Die Einführung von substituierten Schwefel-, Sauerstoff- oder Stickstoffnucleophilen gelingt durch literaturbekannte Methoden der nucleophilen Substitution am Aromaten. Als Abgangsgruppe haben sich bei dieser Substitution Halogenide und Trifluormethansulfonate bewährt. Man arbeitet vorteilhaft in einem dipolar aprotischen

Lösungsmittel, wie zum Beispiel DMF oder TMU bei einer Temperatur zwischen 0°C und dem Siedepunkt des Lösungsmittels, bevorzugt zwischen 80°C und dem Siedepunkt des Lösungsmittels. Als Säurefänger dient vorteilhaft ein Alkali- oder Erdalkalisalz mit einem Anion hoher Basizität und geringer Nucleophilie, wie zum Beispiel K₂CO₃.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Heteroaroylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 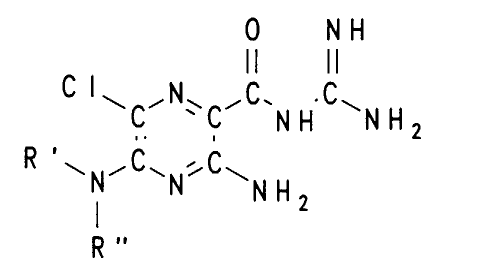 Amilorid: R',R" = H
Dimethylamilorid: R',R" = CH₃
Ethylisopropylamilorid: R' = C₂H₅, R" = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034) werden Benzoylguanidine vorgeschlagen, in welchen aber die Substituenten nicht die nach der vorliegenden Erfindung beanspruchten Bedeutungen haben.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na ⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

Analog der in den Ausführungsbeispielen angegebenen Vorschriften können die nachfolgend aufgeführten erfindungsgemäßen Verbindungen der Formel I bzw. deren physiologisch verträglichen Salze hergestellt werden:

### Liste der Abkürzungen:

- MeOH: Methanol
- DMF: N,N-Dimethylformamid
- TMU: N,N,N',N'-Tetramethylharnstoff
- NBS: N-Bromsuccinimid
- AIBN: α,α-Azo-bis-isobutyronitril
- El: electron impact
- DCI: Desorption-Chemical Ionisation
- RT: Raumtemperatur
- EE: Ethylacetat (EtOAc)
- DIP: Diisopropylether
- MS: Massenspektrum
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-lonisation
- Me: Methyl
- Et: Ethyl
- Bn: Benzyl
- ZNS: Zentralnervensystem
- Brine: gesättigte wäßrige NaCI-Lösung

### Experimenteller Teil

### Allgemeine Vorschrift zur Herstellung von Acyl-guanidinen (I) Variante A: aus Carbonsäuren (II, L = OH)

1.0 eq. des Carbonsäurederivates der Formel II löst bzw. suspendiert man in wasserfreiem THF (5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotavapor) ab, versetzt mit Wasser, stellt mit 2N HCI auf pH 6 bis 7 und filtriert das entsprechende Acyl-guanidin (Formel I) ab. Die so erhaltenen Acyl-guanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Acyl-guanidinen (1)

### Variante B: aus Carbonsäure-alkylestern (II, L = O-Alkyl)

1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: Chinolin-2-carbonsäureguanidid-dihydrochlorid

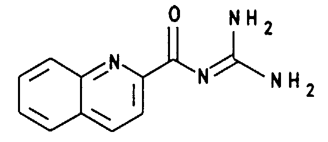

Wurde gemäß Variante A aus Chinolin-2-carbonsäure dargestellt.
MS (ES): 215 (M + 1)
mp : > 250°C

### Beispiel 2: Chinolin-3-carbonsäureguanidid-dihydrochlorid

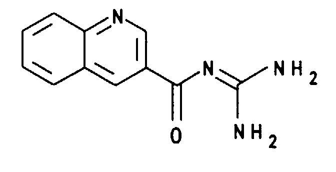

Wurde gemäß Variante A aus Chinolin-3-carbonsäure dargestellt.
MS (ES): 215 (M + 1)
mp : 217°C

### Beispiel 3: Chinolin-6-carbonsäureguanidid

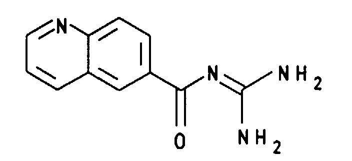

a) 6-Trifluormethylsulfonyloxy-chinolin
   1.5 g 6-Hydroxychinolin wird in 60 ml CH₂Cl₂ gelöst und bei -30°C 1.4 ml 2,6-Lutidin, 250 mg 4-Dimethylaminopyridin und 2.0 ml
   Trifluormethansulfonsäureanhydrid zugegeben. Anschließend wird auf RT erwärmt und 1.5 h nachgerührt. Das Reaktionsgemisch wird auf 100 ml gesättigte wäßrige NaHCO₃-Lösung gegossen und 3 x mit 150 ml Essigester extrahiert. Über Na₂SO₄ wird getrocknet, das Solvens im Vakuum entfernt und mit EE/Hep 2 : 1 chromatographiert. Man erhält 2.0 g farbloses Öl.
   R_{f} (MTB) = 0.59
   MS (DCl) : 278 (M + H)⁺
b) Chinolin-6-carbonsäure-methylester
   2.0 g 6-Trifluormethylsulfonyloxy-chinolin werden in 7 ml MeOH sowie 14 ml DMF gelöst und 2.0 ml Triethylamin sowie 48 mg Palladium(II)acetat und 88 mg 1,3-Bis-(diphenylphosphino)propan zugegeben. Die Luft im Reaktionsgefäß wird durch CO-Gas ersetzt und 2 h bei 70°C gerührt. Das Gemisch wird in 100 ml gesättigte wäßrige NaHCO₃-Lösung gegossen und 3 x mit 150 ml EE extrahiert. Über Na₂SO₄ wird getrocknet, das Solvens im Vakuum entfernt und mit MTB/DIP 1 : 1 chromatographiert. Man erhält 480 mg farbloser Kristalle;
   mp 86°C.
   R_{f} (MTB) = 0.43
   MS (DCl) : 188 (M + H)⁺
c) Chinolin-6-carbonsäureguanidid
   450 mg Chinolin-6-carbonsäure-methylester werden nach Variante B in 410 mg Chinolin-6-carbonsäureguanidid umgewandelt. mp (Dihydrochlorid) > 270°C.
   R_{f} (EE/MeOH 5:1) = 0.13
   MS (DCl) : 215 (M + H)⁺

### Beispiel 4: Isochinolin-6-carbonsäureguanidid

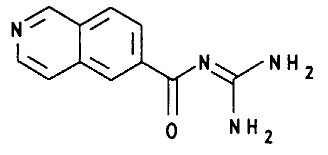

a) Isochinolin-6-carbaldehyd
   1.4 g 6-Methylisochinolin, 4.0 g SeO₂ und 1.7 g K₂CO₃ werden in 40 ml Pyridin 7 d zum Rückfluß erhitzt. Das Reaktionsgemisch wird in 100 ml gesättigte wäßrige Na₂CO₃-Lösung gegossen und 3 x mit 100 ml EE extrahiert. Über Na₂SO₄ wird getrocknet, das Solvens im Vakuum entfernt und mit MTB chromatographiert. Man erhält 290 mg farbloses Öl.
   R_{f} (MTB) = 0.28
   MS (DCl) : 158 (M + H)⁺
b) Isochinolin-6-carbonsäure-methylester
   290 mg Isochinolin-6-carbaldehyd werden in 40 ml MeOH gelöst und zunächst 450 mg NaCN, dann 210 µl Eisessig und schließlich 3.5 g MnO₂ zugegeben. 3 d wird bei RT gerührt, vom Niederschlag abfiltriert und das Filtrat in eine Lösung von 15 g FeSO₄ in 150 ml Wasser gegossen. Anschließend wird mit Na₂CO₃-Lösung auf pH = 9 eingestellt, der Niederschlag abfiltriert und 3 x mit 150 ml EE extrahiert. Über Na₂SO₄ wird getrocknet und das Solvens im Vakuum entfernt. Man erhält 190 mg farbloses Öl, das ohne weitere Reinigung umgesetzt wird.
   R_{f} (MTB) = 0.40
   MS (DCl) : 188 (M + H)⁺
c) Isochinolin-6-carbonsäureguanidid
   180 mg Isochinolin-6-carbonsäure-methylester werden nach Variante B in 45 mg Isochinolin-6-carbonsäureguanidid umgewandelt.
   mp (Dihydrochlorid) > 270°C
   R_{f} (EE/MeOH 5:1) = 0.20
   MS (EI) : 215 (M + H)⁺

### Beispiel 5: 2-Naphthoesäureguanidid-hydrochlorid

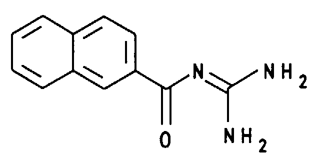

Wurde gemäß Variante A aus Naphthalin-2-carbonsäure dargestellt.
MS (ES): 250 (M + 1)

### Beispiel 6: Isochinolin-3-carbonsäureguanidid

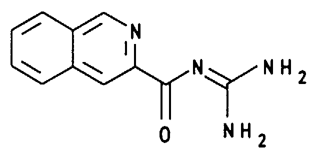

Wurde gemäß Variante A aus Isochinolin-3-carbonsäure dargestellt. MS (ES): 215 (M + 1) mp : 153°C

### Beispiel 7: 4-Hydroxy-5,8-difluor-chinolin-3-carbonsäureguanidid

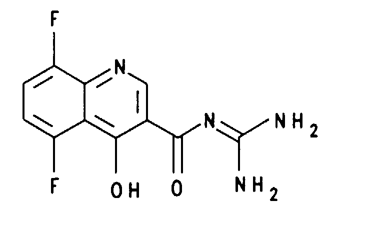

Wurde gemäß Variante B aus dem entsprechenden Ethylester dargestellt.
MS (ES): 284 (M + 1)
mp : > 230°C

### Beispiel 8: 6-Chlor-2-methyl-chinolin-3-carbonsäureguanidid-hydrochlorid

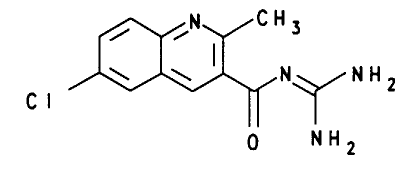

a) 6-Chlor-2-methyl-chinolin-3-carbonsäureethylester
   Wurde gemäß eines literaturbekannten Verfahrens [David R. Adams, Trina Colman de Saizarbitoria, Synthetic Communications 17 (14), 1647-1653 (1987) ] aus 4-Chloranilin und Acetylessigsäureethylester hergestellt.
b) 6-Chlor-2-methyl-chinolin-3-carbonsäureguanidid-hydrochlorid
   Wurde gemäß Variante B aus 6-Chlor-2-methyl-chinolin-3-carbonsäureethylester dargestellt.
   MS (ES): 263 (M + 1)
   mp : 213°C

### Beispiel 9: 4-Hydroxy-7-trifluormethyl-chinolin-2-carbonsäureguanidid-hydrochlorid

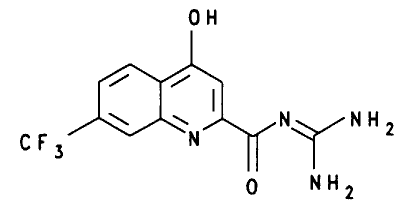

Wurde gemäß Variante B aus 4-Hydroxy-7-trifluormethyl-chinolin-2-carbonsäureester dargestellt.
MS (ES): 299 (M + 1)
mp : 142 - 145°C

### Beispiel 10: 4,8-Dihydroxy-chinolin-3-carbonsäureguanidid-hydrochlorid

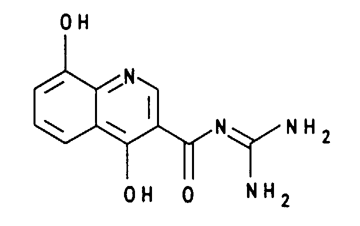

Wurde gemäß Variante B aus 4,8-Dihydroxy-chinolin-3-carbonsäureester dargestellt.
MS (ES): 247 (M + 1)
mp : 255 - 260°C

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na ⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l: 140 NaCI, 3 KCI, 150 Sucrose, 0,1 Ouabain, 20 Tris-hydroxymethyl-aminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amiloridhemmbare Natrium-lnflux ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

Inhibition des Na⁺/H⁺-Exchangers:

| Beispiel | IC₅₀ [µmol/l] |
|---|---|
| 1 | 2-3 |
| 2 | 1.2 |
| 3 | 3-5 |
| 4 | 10 |
| 8 | <1 |

## Patentansprüche

1. Bizyklische Heteroaroylguanidine der Formel I 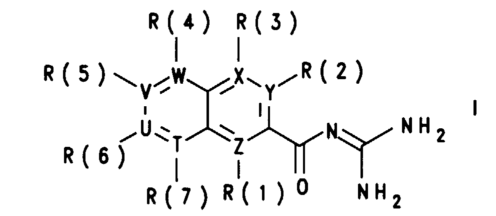 worin bedeuten:
T, U, V, W, X, Y und Z unabhängig voneinander Stickstoff oder Kohlenstoff;
jedoch mit der Einschränkung,
daß nur eine der Positionen T, U, V, W, X, Y und Z Stickstoff ist,
und
daß T, U, V, W, X, Y und Z keinen Substituenten tragen, wenn sie Stickstoff sind,
und
daß T, U, V, W, X, Y und Z nicht gleichzeitig Kohlenstoff sind;
und
daß R(3), R(4), R(5), R(6) und R(7) nicht alle gleichzeitig Wasserstoff sind, wenn
a) T, U, V, W, X und Z gleich Kohlenstoff und Y gleich Stickstoff sind,
oder
b) T, U, V, W, Y und Z gleich Kohlenstoff und X gleich Stickstoff sind;
R(1) und R(2) unabhängig voneinander Wasserstoff, F, Cl, Br, I, (C₁-C₃)-Alkyl, (C₁-C₃)-Perfluoralkyl, OR(8) oder NR(8)R(9);
R(8) und R(9) unabhängig voneinander Wasserstoff oder (C₁-C₃)-Alkyl;
oder
R(8) und R(9) gemeinsam 4 oder 5 Methylengruppen, von denen eine CH₂-Gruppe durch Sauerstoff, Schwefel, NH, N-CH₃ oder N-Benzyl ersetzt sein kann,
R(3), R(4), R(5), R(6) und R(7) unabhängig voneinander Wasserstoff, F, Cl, Br, I, -C≡N, CF₃, CH₃SO₂ oder CH₃CO;
oder
R(3), R(4), R(5), R(6) und R(7) unabhängig voneinander (C₁-C₄)-Alkyl oder -C_{aI}H_{2aI}R(18);
al Null oder 1;
R(18) Phenyl, welches nicht substituiert oder substituiert ist mit 1 - 3 Substituenten ausgewählt aus der Gruppe bestehend aus F, Cl, CF₃, Methyl, Methoxy und NR(19a)R(19b);
R(19a) und R(19b) H, CH₃ oder CF₃;
oder
R(3), R(4), R(5), R(6) und R(7) unabhängig voneinander (C₁-C₉)-Heteroaryl, das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(3), R(4), R(5), R(6) und R(7) unabhängig voneinander SR(29), -OR(30), -NR(31)R(32) oder -CR(33)R(34)R(35);
R(29), R(30), R(31) und R(33) unabhängig voneinander -CₐH₂ₐ-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null, 1 oder 2;
R(32), R(34) und R(35) unabhängig voneinander wie R(29) definiert oder Wasserstoff, CH₃ oder CF₃;
oder
R(3), R(4), R(5), R(6) und R(7) unabhängig voneinander NR(84a)R(85), OR(84b) oder -CₙH₂ₙ-R(84d);
n Null oder 1;
R(84d) Phenyl, welches nicht substituiert ist oder substituiert mit 1 - 3 Substituenten ausgewählt aus der Gruppe bestehend aus F, Cl, CF₃, Methyl, Methoxy und NR(16)R(17);
R(16) und R(17) Wasserstoff oder CH₃;
R(84a), R(84b) und R(85) unabhängig voneinander H, (C₁-C₄)-Alkyl, CF₃ oder (CH₂)ₐₓ-R(84g);
ax Null oder 1;
R(84g) Phenyl, welches nicht substituiert ist oder substituiert mit 1 - 3 Substituenten ausgewählt aus der Gruppe bestehend aus F, Cl, CF₃, Methyl, Methoxy und NR(84u)R(84v);
R(84u) und R(84v) Wasserstoff oder CH₃;
oder
R(84a) und R(85) gemeinsam 4 oder 5 Methylengruppen, von denen eine CH₂-Gruppe durch Sauerstoff, Schwefel, NH, N-CH₃ oder N-Benzyl ersetzt sein kann,
sowie deren pharmazeutisch verträgliche Salze.

2. Verbindung der Formel I nach Anspruch 1, in der bedeuten:
T, U, V, W, Y und Z Kohlenstoff;
X Stickstoff;
jedoch mit der Einschränkung,
daß X keinen Substituenten trägt,
und
daß R(4), R(5), R(6) und R(7) nicht alle gleichzeitig Wasserstoff sind;
R(1) und R(2) unabhängig voneinander Wasserstoff, F, Cl, CH₃, CF₃, OH, OCH₃, NH₂;
R(4), R(5), R(6) und R(7) unabhängig voneinander Wasserstoff, F, Cl, Br, I, -C≡N, CF₃, CH₃SO₂ oder CH₃CO,
mit der Einschränkung, daß sie nicht alle gleichzeitig Wasserstoff sind;
oder
R(4), R(5), R(6) und R(7) unabhängig voneinander (C₁-C₄)-Alkyl oder -C_{aI}H_{2aI}R(18);
al Null oder 1;
R(18) Phenyl, welches nicht substituiert oder substituiert ist mit 1 - 3 Substituenten ausgewählt aus der Gruppe bestehend aus F, Cl, CF₃, Methyl, Methoxy und NR(19a)R(19b);
R(19a) und R(19b) H, CH₃ oder CF₃;
oder
R(4), R(5), R(6) und R(7) unabhängig voneinander SR(29) oder -OR(30);
R(29) und R(30) unabhängig -CₐH₂ₐ-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
a Null oder 1;
oder
R(4), R(5), R(6) und R(7) unabhängig voneinander NR(84a)R(85), OR(84b) oder -CₙH₂ₙ-R(84d);
n Null oder 1;
R(84d) Phenyl, welches nicht substituiert ist oder substituiert mit 1 - 3 Substituenten ausgewählt aus der Gruppe bestehend aus F, Cl, CF₃, Methyl, Methoxy und NR(16)R(17);
R(16) und R(17) Wasserstoff oder CH₃;
R(84a), R(84b) und R(85) unabhängig voneinander H, (C₁-C₄)-Alkyl, CF₃ oder (CH₂)ₐₓ-R(84g);
ax Null oder 1;
R(84g) Phenyl, welches nicht substituiert ist oder substituiert mit 1 - 3 Substituenten ausgewählt aus der Gruppe bestehend aus F, Cl, CF₃, Methyl, Methoxy und NR(84u)R(84v);
R(84u) und R(84v) Wasserstoff oder CH₃.

3. Verfahren zum Herstellen einer Verbindung I nach Anspruch 1, dadurch gekennzeichnet, daß man eine Verbindung der Formel II 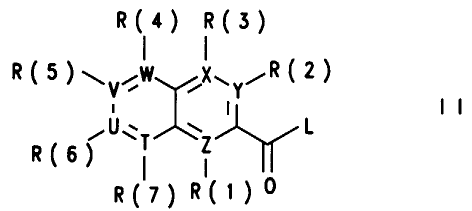 in der R(1) bis R(7) und T, U, V, W, X, Y und Z wie in Anspruch 1 definiert sind und worin L für eine leicht nucleophil substituierbare Fluchtgruppe steht, mit Guanidin umsetzt.

4. Verwendung einer Verbindung I nach Anspruch 1 zur Herstellung eines Medikaments zu Behandlung von Arrhythmien.

5. Verwendung einer Verbindung I nach Anspruch 1 zur Herstellung eines Medikaments zur Behandlung oder Prophylaxe des Herzinfarkts.

6. Verwendung einer Verbindung I nach Anspruch 1 zur Herstellung eines Medikaments zur Behandlung oder Prophylaxe der Angina Pectoris.

7. Verwendung einer Verbindung I nach Anspruch 1 zur Herstellung eines Medikaments zur Behandlung oder Prophylaxe von ischämischen Zuständen des Herzens.

8. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

9. Verwendung einer Verbindung I nach Anspruch 1 zur Herstellung eines Medikaments zur Behandlung oder Prophylaxe von ischämischen Zuständen peripherer Organe und Gliedmaßen.

10. Verwendung einer Verbindung I nach Anspruch 1 zur Herstellung eines Medikaments zur Behandlung von Schockzuständen.

11. Verwendung einer Verbindung I nach Anspruch 1 zur Herstellung eines Medikaments zum Einsatz bei chirurgischen Operationen und Organtransplantationen.

12. Verwendung einer Verbindung I nach Anspruch 1 zur Herstellung eines Medikaments zur Konservierung und Lagerung von Transplantaten für chirurgische Maßnahmen.

13. Verwendung einer Verbindung I nach Anspruch I zur Herstellung eines Medikaments zur Behandlung von Krankheiten, bei denen die Zellproliferation eine primäre oder sekundäre Ursache darstellt.

14. Heilmittel, enthaltend eine wirksame Menge einer Verbindung I nach einem der Ansprüche 1 oder 2.

## Claims

1. A bicyclic heteroaroylguanidine of the formula I 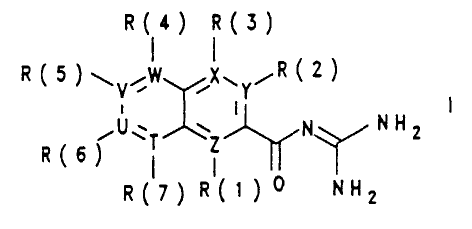 in which:
T, U, V, W, X, Y and Z are, independently of each other, nitrogen or carbon;
with, however, the restriction
that only one of the positions T, U, V, W, X, Y and
Z is nitrogen
and
that T, U, V, W, X, Y and Z do not carry any substituent when they are nitrogen,
and
that T, U, V, W, X, Y and Z are not simultaneously carbon;
and
that R(3), R(4), R(5), R(6) and R(7) are not all simultaneously hydrogen, when
a) T, U, V, W, X and Z are carbon and Y is nitrogen,
or
b) T, U, V, W, Y and Z are carbon and X is nitrogen;
R(1) and R(2), are independently of each other, hydrogen, F, Cl, Br, I, (C₁-C₃)-alkyl (C₁-C₃)-perfluoroalkyl, OR(8) or NR(8)R(9);
R(8) and R(9) are, independently of each other, hydrogen or (C₁-C₃)-alkyl;
or
R(8) and R(9) are together 4 or 5 methylene groups of which one CH₂ group can be replaced by oxygen, sulfur, NH, N-CH₃ or N-benzyl,
R(3), R(4), R(5), R(6) and R(7) are, independently of each other, hydrogen, F, Cl, Br, I, -C≡N, CF₃, CH₃SO₂ or CH₃CO;
or
R(3), R(4), R(5), R(6) and R(7) are, independently of each other, (C₁-C₄)-alkyl or -CₐₗH₂ₐₗR(18);
al is zero or 1;
R(18) is phenyl which is not substituted or is substituted by 1-3 substituents selected from the group consisting of F, Cl, CF₃, methyl, methoxy and NR(19a)R(19b);
R(19a) and R(19b) are H, CH₃ or CF₃;
or
R(3), R(4), R(5), R(6) and R(7) are, independently of each other, (C₁-C₉)-heteroaryl which is linked via C or N and which is unsubstituted or is substituted by 1-3 substituents selected from the group consisting of F, Cl, CF₃, CH₃, methoxy, hydroxy, amino, methylamino and dimethylamino;
or
R(3), R(4), R(5), R(6) and R(7) are, independently of each other, SR(29), -OR(30), -NR(31)R(32) or -CR(33)R(34)R(35);
R(29), R(30), R(31) and R(33) are, independently, of each other -CₐH₂ₐ-(C₁-C₉)-heteroaryl which is unsubstituted or is substituted by 1-3 substituents selected from the group consisting of F, Cl, CF₃, CH₃, methoxy, hydroxyl, amino, methylamino and dimethylamino;
a is zero, 1 or 2;
R(32), R(34) and R(35) are, independently of each other, as defined in R(29) or hydrogen, CH₃ or CF₃;
or
R(3), R(4), R(5), R(6) and R(7) are, independently of each other, NR(84a)R(85), OR(84b) or -CₙH₂ₙ-R(84d);
n is zero or 1;
R(84d) is phenyl which is not substituted or is substituted by 1-3 substituents selected from the group consisting of F, Cl, CF₃, methyl, methoxy and NR(16)R(17);
R(16) and R(17) are hydrogen or CH₃;
R(84a), R(84b) and R(85) are, independently of each other, H, (C₁-C₄)-alkyl, CF₃ or (CH₂)ₐₓ-R(84g);
ax is zero or 1;
R (84g) is phenyl which is not substituted or is substituted by 1-3 substituents selected from the group consisting of F, Cl, CF₃, methyl, methoxy and NR(84u)R(84v);
R(84u) and R(84v) are hydrogen or CH₃;
or
R(84a) and R(85) are together 4 or 5 methylene groups of which one CH₂ group can be replaced by oxygen, sulfur, NH, N-CH₃ or N-benzyl,
and the pharmaceutically tolerated salts thereof.

2. A compound of the formula I as claimed in claim 1, in which:
T, U, V, W, Y and Z are carbon;
X is nitrogen;
with, however, the restriction,
that X does not carry any substituent,
and
that R(4), R(5), R(6) and R(7) are not all simultaneously hydrogen;
R(1) and R(2) are, independently of each other, hydrogen, F, Cl, CH₃, CF₃OH, OCH₃ or NH₂;
R(4), R(5), R(6) and R(7) are, independently of each other, hydrogen, F, Cl, Br, I, -C≡N, CF₃, CH₃SO₂ or CH₃CO,
with the restriction that they are not all simultaneously hydrogen;
or
R(4), R(5), R(6) and R(7) are, independently of each other, (C₁-C₄)-alkyl or CₐₗH₂ₐₗR(18) ;
al is zero or 1;
R(18) is phenyl which is not substituted or is substituted by 1-3 substituents selected from the group consisting of F, Cl, CF₃, methyl, methoxy and NR(19a)R(19b);
R(19a) and R(19b) are H, CH₃ or CF₃;
or
R(4), R(5), R(6) and R(7) are, independently of each other, SR(29) or -OR(30);
R(29) and R(30) are, independently, -CₐH₂ₐ-(C₁-C₉)-heteroaryl, which is unsubstituted or substituted by 1-3 substituents selected from the group consisting of F, Cl, CF₃, CH₃, methoxy, hydroxyl, amino, methylamino and dimethylamino;
a is zero or 1;
or
R(4), R(5), R(6) and R(7) are, independently of each other, NR(84a)R(85), OR(84b) or -CₙH₂ₙ-R(84d);
n is zero or 1;
R(84d) is phenyl which is not substituted or is substituted by 1-3 substituents selected from the group consisting of F, Cl, CF₃, methyl, methoxy and NR(16)R(17);
R(16) and R(17) are hydrogen or CH₃;
R(84a), R(84b) and R(85) are, independently of each other, H, (C₁-C₄)-alkyl, CF₃ or (CH₂)ₐₓ-R(84g);
ax is zero or 1;
R(84g) is phenyl which is not substituted or is substituted by 1-3 substituents selected from the group consisting of F, Cl, CF₃, methyl, methoxy and NR(84u)R(84v);
R(84u) and R(84v) are hydrogen or CH₃.

3. A process for preparing a compound I as claimed in claim 1, wherein a compound of the formula II 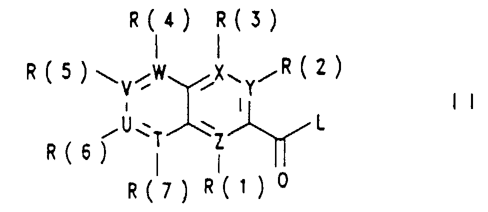 in which R(1) to R(7) and T, U, V, W, X, Y and Z are defined as in claim 1 and in which L is a leaving group which can readily be substituted nucleophilically, is reacted with guanidine.

4. Use of a compound I as claimed in claim 1 for preparing a medicament for the treatment of arrhythmias.

5. Use of a compound I as claimed in claim 1 for preparing a medicament for the treatment or prophylaxis of cardiac infarction.

6. Use of a compound I as claimed in claim 1 for preparing a medicament for the treatment or prophylaxis of angina pectoris.

7. Use of a compound I as claimed in claim 1 for preparing a medicament for the treatment or prophylaxis of ischemic conditions of the heart.

8. Use of a compound I as claimed in claim 1 for preparing a medicament for the treatment or prophylaxis of ischemic conditions of the peripheral and central nervous system and of stroke.

9. Use of a compound I as claimed in claim 1 for preparing a medicament for the treatment or prophylaxis of ischemic conditions of peripheral organs and limbs.

10. Use of a compound I as claimed in claim 1 for preparing a medicament for the treatment of shock conditions.

11. Use of a compound I as claimed in claim 1 for preparing a medicament for employment in surgical operations and organ transplantations.

12. Use of a compound I as claimed in claim 1 for preparing a medicament for the preservation and storage of transplants for surgical procedures.

13. Use of a compound I as claimed in claim 1 for preparing a medicament for the treatment of diseases in which cell proliferation represents a primary or secondary cause.

14. A medicine, containing an effective quantity of a compound I as claimed in either of claims 1 or 2.

## Revendications

1. Hétéroaroylguanidines bicycliques de formule I 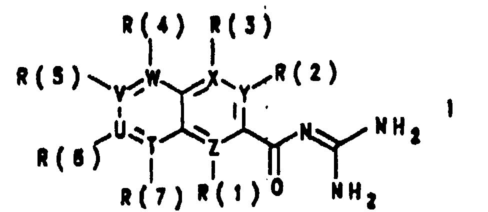 dans laquelle
T, U, V, W, X, Y et Z représentent, indépendamment les uns des autres, un atome d'azote ou de carbone,
mais avec la restriction
que seule une des positions T, U, V, W, X, Y et Z soit un atome d'azote,
et
que T, U, V, W, X, Y et Z ne portent pas de substituants lorsqu'ils représentent un atome d'azote,
et
que T, U, V, W, X, Y et Z ne soient pas simultanément des atomes de carbone,
et
que R(3), R(4), R(5), R(6) et R(7) ne soient pas tous simultanément des atomes d'hydrogène lorsque
a) T, U, V, W, X et Z représentent des atomes de carbone et Y représente un atome d'azote,
ou
b) T, U, V, W, Y et Z représentent des atomes de carbone et X représente un atome d'azote;
R(1) et R(2) représentent, indépendamment l'un de l'autre, F, CI, Br, I ou un groupe alkyle en C₁-C₃, perfluoroalkyle en C₁-C₃, OR(8) ou NR(8)R(9);
R(8) et R(9) représentent, indépendamment l'un de l'autre, un atome d'hydrogène ou un groupe alkyle en C₁-C₃;
ou
R(8) et R(9) représentent ensemble 4 ou 5 groupes méthylène, dont un groupe CH₂ peut être remplacé par un atome d'oxygène ou de soufre ou par un groupe NH, N-CH₃ ou N-benzyle;
R(3), R(4), R(5), R(6) et R(7) représentent, indépendamment les uns des autres, un atome d'hydrogène, F, CI, Br, I, -C≡N, CF₃, CH₃SO₂ ou CH₃CO;
ou
R(3), R(4), R(5), R(6) et R(7) représentent, indépendamment les uns des autres, un groupe alkyle en C₁-C₄ ou CₐₗH₂ₐₗR(18),
al représentant zéro ou 1,
R(18) représentant un groupe phényle qui n'est pas substitué ou porte 1-3 substituants choisis dans l'ensemble constitué par F, CI, CF₃, les groupes méthyle, méthoxy et NR(19a)R(19b),
R(19a) et R(19b) représentant H, CH₃ ou CF₃;
ou
R(3), R(4), R(5), R(6) et R(7) représentent, indépendamment les uns des autres, un groupe hétéroaryle en C₁-C₉ qui est relié par C ou N et qui n'est pas substitué ou porte 1-3 substituants choisis dans l'ensemble constitué par F, CI, CF₃, CH₃, les groupes méthoxy, hydroxyle, amino, méthylamino et diméthylamino;
ou
R(3) , R(4), R(5) , R(6) et R(7) représentent, indépendamment les uns des autres, SR(29) , -OR(30), -NR(31)R(32) ou -CR(33)R(34)R(35) ;
R(29), R(30), R(31) et R(33) représentent, indépendamment les uns des autres, un groupe -CₐH₂ₐ-hétéroaryle(C₁-C₉) qui n'est pas substitué ou porte 1-3 substituants choisis dans l'ensemble constitué par F, CI, CF₃, CH₃, les groupes méthoxy, hydroxyle, amino, méthylamino et diméthylamino;
a est zéro, 1 ou 2;
R(32), R(34) et R(35) indépendamment les uns des autres, sont définis comme R(29) ou représentent un atome d'hydrogène, CH₃ ou CF₃;
ou
R(3), R(4), R(5), R(6) et R(7) représentent, indépendamment les uns des autres, NR(84a)R(85), OR(84b) ou -CₙH₂ₙ-R(84d);
n représente zéro ou 1,
R(84d) représente un groupe phényle qui n'est pas substitué ou porte 1-3 substituants choisis dans l'ensemble constitué par F, CI, CF₃, les groupes méthyle, méthoxy et NR(16)R(17),
R(16) et R(17) représentant H ou CH₃;
R(84a), R(84b) et R(85) représentent, indépendamment les uns des autres, H ou un groupe alkyle en C₁-C₄, CF₃ ou (CH₂)ₐₓR(84g),
ax représente zéro ou 1;
R(84g) représente un groupe phényle qui n'est pas substitué ou porte 1-3 substituants choisis dans l'ensemble constitué par F, Cl, CF₃, les groupes méthyle, méthoxy et NR(84u)R(84v),
R(84u) et R(84v) représentant un atome d'hydrogène ou CH₃;
ou
R(84a) et (R85) représentent ensemble 4 ou 5 groupes méthylène, dont un groupe CH₂ peut être remplacé par un atome d'oxygène ou de soufre ou par un groupe NH, N-CH₃ ou N-benzyle;
ainsi que leurs sels pharmaceutiquement acceptables.

2. Composé de formule I selon la revendication 1, dans lequel:
T, U, V, W, Y et Z représentent des atomes de carbone,
X représente un atome d'azote,
mais avec la restriction
que X ne porte aucun substituant,
et
que R(4), R(5), R(6) et R(7) ne soient pas tous simultanément des atomes d'hydrogène
R(1) et R(2) représentent, indépendamment l'un de l'autre, F, CI, CH₃, CF₃, OH, OCH₃, NH₂;
R(4), R(5), R(6) et R(7) représentent, indépendamment les uns des autres, un atome d'hydrogène, F, CI, Br, I, -C≡N, CF₃, CH₃SO₂ ou CH₃CO,
avec la restriction qu'ils ne soient pas tous simultanément des atomes d'hydrogène;
ou
R(4) , R(5), R(6) et R(7) représentent, indépendamment les uns des autres, un groupe alkyle en C₁-C₄ ou -CₐₗH₂ₐₗR(18)
al représentant zéro ou 1,
R(18) représentant un groupe phényle qui n'est pas substitué ou porte 1-3 substituants choisis dans l'ensemble constitué par F, CI, CF₃, les groupes méthyle, méthoxy et NR(19a)R(19b),
R(19a) et R(19b) représentant H, CH₃ ou CF₃;
ou
R(4), R(5), R(6) et R(7) représentent, indépendamment les uns des autres, SR(29) ou -OR(30),
R(29) et R(30) représentent, indépendamment l'un de l'autre, un groupe -CₐH₂ₐ-hétéroaryle(C₁-C₉) qui n'est pas substitué ou porte 1-3 substituants choisis dans l'ensemble constitué par F, CI, CF₃, CH₃, les groupes méthoxy, hydroxyle, amino, méthylamino et diméthylamino;
a est zéro ou 1;
ou
R(4), R(5), R(6) et R(7) représentent, indépendamment les uns des autres, NR(84a)R(85), OR(84b) ou -CₙH₂ₙ-R(84d);
n représente zéro ou 1,
R(84d) représente un groupe phényle qui n'est pas substitué ou porte 1-3 substituants choisis dans l'ensemble constitué par F, CI, CF₃, les groupes méthyle, méthoxy et NR(16)R(17),
R(16) et R(17) représentant un atome d'hydrogène ou CH₃;
R(84a), R(84b) et R(85) représentent, indépendamment les uns des autres, H ou un groupe alkyle en C₁-C₄, CF₃ ou (CH₂)ₐₓR(84g),
ax représente zéro ou 1;
R(84g) représente un groupe phényle qui n'est pas substitué ou porte 1-3 substituants choisis dans l'ensemble constitué par F, Cl, CF₃, les groupes méthyle, méthoxy et NR(84u)R(84v),
R(84u) et R(84v) représentant un atome d'hydrogène ou CH₃.

3. Procédé pour la préparation d'un composé I selon la revendication 1, caractérisé en ce que l'on fait réagir avec de la guanidine un composé de formule II 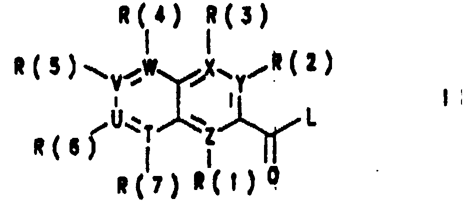 dans laquelle R(1) à R(7) et T, U, V, W, X et Z sont tels que définis dans la revendication 1, et où L représente un groupe partant pouvant être aisément remplacé par substitution nucléophile.

4. Utilisation d'un composé I selon la revendication 1, pour la fabrication d'un médicament destiné au traitement d'arythmies.

5. Utilisation d'un composé I selon la revendication 1, pour la fabrication d'un médicament destiné au traitement ou à la prophylaxie de l'infarctus du myocarde.

6. Utilisation d'un composé I selon la revendication 1, pour la fabrication d'un médicament destiné au traitement ou à la prophylaxie de l'angine de poitrine.

7. Utilisation d'un composé I selon la revendication 1, pour la fabrication d'un médicament destiné au traitement ou à la prophylaxie d'états ischémiques du coeur.

8. Utilisation d'un composé I selon la revendication 1, pour la fabrication d'un médicament destiné au traitement ou à la prophylaxie d'états ischémiques du système nerveux périphérique et central et de l'apoplexie cérébrale.

9. Utilisation d'un composé I selon la revendication 1, pour la fabrication d'un médicament destiné au traitement ou à la prophylaxie d'états ischémiques d'organes périphériques et des membres.

10. Utilisation d'un composé I selon la revendication 1, pour la fabrication d'un médicament destiné au traitement d'états de choc.

11. Utilisation d'un composé I selon la revendication 1, pour la fabrication d'un médicament destiné à l'utilisation dans des opérations chirurgicales et des greffes d'organes.

12. Utilisation d'un composé I selon la revendication 1, pour la fabrication d'un médicament destiné à la conservation et au stockage de transplants pour des opérations chirurgicales.

13. Utilisation d'un composé I selon la revendication 1, pour la fabrication d'un médicament destiné au traitement de maladies dans lesquelles la prolifération cellulaire représente une cause primaire ou secondaire.

14. Médicament, caractérisé par une teneur efficace en un composé de formule I selon la revendication 1 ou 2.
